# EUROPEAN PATENT SPECIFICATION

(11) **EP 1 635 850 B1**
(45) Date of publication and mention of the grant of the patent: **13.08.2008**
(21) Application number: 04742985.7
(22) Date of filing: 21.06.2004
(51) Int. Cl.: A61K 33/38, A61L 15/00, A61P 17/02, A61K 31/44

(54) **ANTIMICROBIAL SILVER COMPRISING SILVER**
ANTIMIKROBIELLE ZUSAMMENSETZUNG ENTHALTEND SILBER
ANTIMICROBIEN A L'ARGENT

(30) Priority: 20.06.2003 GB 0314453; 04.08.2003 US 491990 P
(43) Date of publication of application: 22.03.2006
(73) Proprietor: Johnson & Johnson Medical Ltd., Gargrave, Skipton BD23 3RX (GB)
(72) Inventor: TROTTER, Patrick, Meanwood, Leeds LS6 4RD (GB); JAMPANI, Hanuman, Flemington, NJ 08822 (US); MITSCHER, Lester, Lawrence, KS 66047 (US); PILLAI, Segaran, Laurel, MD 2702 (US)
(74) Representative: James, Anthony Christopher W.P.
(86) International application number: PCT/GB2004/002631
(87) International publication number: WO 2004/112805

(56) References cited:
- EP-A- 1 153 622
- WO-A-01/26627
- WO-A-94/21288
- WO-A-03/028762
- GB-A- 2 206 495
- RU-C- 2 138 183
- US-A- 5 744 151

## Description

The present invention relates to antimicrobial compositions comprising silver in combination with an inhibitor of microbial silver resistance.

The antimicrobial effect of silver has been known for centuries. However, the precise mode of action of silver salts in killing microbes is yet to be established. It is known that silver salts bind with particular avidity to DNA and RNA. Silver salts also bind with particular strength to a variety of organic molecules such as: carboxylic acids, thiols, phenols, amines, phosphates and halogenated compounds. Following binding to proteins, those with enzymic activity are usually deactivated. The oxidative-reductive powers of silver and silver salts must also be reckoned with.

Metallic silver is an effective antimicrobial, whether in the form of thin films, nanoparticles or colloidal silver. Chemical compounds of silver are also useful as antimicrobials. For example, the following complex silver salts are favored for use against sensitive and resistant bacterial strains:

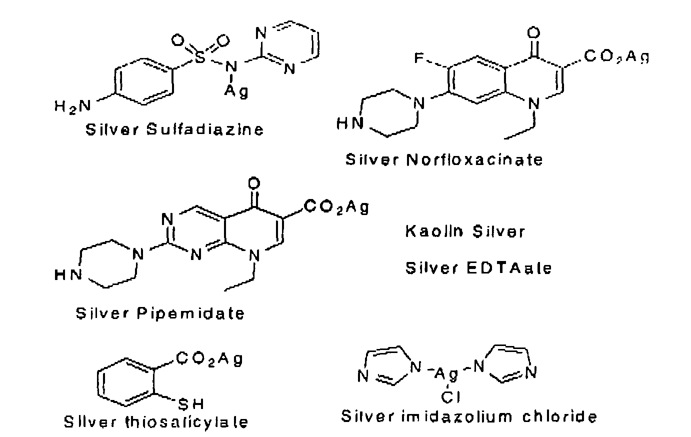

Resistance of certain microbes to silver salts is also known, and is usually attributed either to the presence of a silver-specific binding protein that intercepts the ion, or to two different export proteins. One of the silver export proteins is a plasmid-coded P-type ATPase *(silP)* and the other is a plasmid-coded membrane potential dependent three-polypeptide cation/proton antiporter *(silCBA).*

US 5744151 discloses an antimicrobial composition comprising silver and salicylic acid. The composition has a final concentration of 5% salicylic acid and 0.2% stabilized silver nitrate. Said composition has shown antimicrobial activity on Staphylococcus aureus and Escherichia coli and can be incorporated in different wound dressings.

WO 01/26627 describes a pharmaceutical composition for the treatment of bacterial or fungal infections in a mammal comprising one silver component and phosphatidiylcholine, sphingomyelin, cholesterol, phosphatidylethanolamine or phosphatidylserine.

WO03/028762 describes topical antimicrobial compositions that control or prevent resistance to antimicrobial effectiveness. The compositions comprise an antimicrobial agent in combination with antimutagenic or antioxidant agents that block intrinsic or acquired bacterial resistance. The antimutagenic or antioxidant agents apparently function by preventing free radical damage to bacterial DNA. However, they are unlikely to be effective against mutations due to genetic transfer, for example genetic transfer by viruses or plasmids, which is the most common cause of spread of antibiotic resistance. Accordingly, there is a need for better methods of inhibiting resistance to antimicrobial effectiveness.

It has now been found that certain additives can inhibit or reverse the development of resistance to silver in certain microbes by interacting with the microbial cell wall to disrupt the structure of the cell wall thereby allowing silver into the microbial cell and/or by promoting the transfer of silver through the microbial cell wall and/or by inhibiting the ion pump that transfers silver ions out of the microbial cell through the cell wall.

Accordingly, in a first aspect, the present invention provides an antimicrobial composition comprising silver and fusaric acid which interacts with a microbial cell wall to inhibit microbial silver resistance.

Preferably, the inhibitor substance is a substance that, when tested in accordance with the methods described below, inhibits the development of silver resistance in at least one microorganism selected from the group consisting of *Staphylococcus aureus* (ATCC 13709), *Staphylococcus epidermidis* (various CNS strains), *Klebsiella pneumoniae* (ATCC 10031), *Candida albicans* (ATCC 10231), *Escherichia coli* (ATCC 9637 and AG100), *Pseudomonas aeruginosa* (ATCC 27853 and various PSI strains), *Staphylococcus aureus, Vancomycin resistant Enterococci* (VRE F23232, VRE F23245 and VRE 4030101). Other microorganisms of interest include *Streptococcus Pyogenes,* and certain anaerobic bacteria such as *Bacteroides spp., Prevotella spp, porphyromonas spp, Peptostreptococcus spp,* and *Clostridia spp.* In some embodiments, the inhibitor compound does not have antimicrobial activity against the target microorganism when it is administered alone, i.e. without silver.

The resistance inhibitors are expected to include molecules that can promote the transport of silver across the cell wall, and/or disrupt the cell wall to allow silver into the cell of the microorganisms, and/or disrupt ion pump mechanisms in the cell wall for removing silver from the cell. They may for example function by active transport of the silver ions, or by modifying the permeability properties of the membrane, for example by physical disruption or by modifying the type and nature of phospholipids present in the membrane. Preferably the resistance inhibitors of the present invention are not antimutagenic or antioxidant compounds.

The antimicrobial composition according to the present invention may typically comprise from about 0.01 wt.% to about 5wt.% of the resistance inhibiting compound, based on the dry weight of the composition, for example from about 0.1wt-% to about 3 wt.% of the resistance inhibiting compound.

The term "silver" herein refers to metallic silver and/or compounds of silver. In certain embodiments the silver comprises metallic silver, in which case it may for example be in the form of a thin film or colloidal particles (nanoparticles) as conventionally known in the art. Alternatively or additionally the silver may be present as a chemical compound or complex, such as those listed above in connection with the prior art.

Preferably, the amount of silver in the compositions according to the present invention is from about 0.01 wt% to about 5wt.%, more preferably from about 0.1 wt% to about 2wt.%, and most preferably about 0.1 wt.% to about 1 wt.%, most preferably about 0.3wt.%. Lesser amounts of silver could give insufficient antimicrobial effect. Greater amounts of silver could give rise to antiproliferative effects on wound healing cells.

The silver and the inhibitor compound may be dispersed in or on any conventional vehicle for antimicrobial compositions, such as a liquid, gel, paste, or solid. In certain embodiments the silver and the inhibitor compound may be dispersed in or on a solid support material, and preferably they are applied to a surface of such a material.

For example, the solid support material may a woven fabric, a knitted fabric, a nonwoven fabric, or a freeze-dried or solvent-dried sponge. For example, the solid support material may be a fabric comprising textile filaments that are individually coated with a thin film of silver. Especially suitable support materials in include charcoal cloth materials of the kind described in GB-A-2206495 and GB-A-2167053. Other suitable support materials include oxidized regenerated cellulose cloths, such as SURGICEL cloth available from Johnson & Johnson Medical Limited. Other suitable support materials are freeze-dried sponges comprising for example collagen, ORC, alginates, chitosan, or mixtures thereof. Freeze-dried sponges of this type are described for example in EP-A-1153622.

In a second aspect, the present invention provides a wound dressing comprising an antimicrobial composition according to the present invention.

The wound dressing is preferably in sheet form and comprises an active layer of the material according to the invention. The active layer would normally be the wound contacting layer in use, but in some embodiments it could be separated from the wound by a liquid-permeable top sheet. Preferably, the area of the active layer is from about 1 cm² to about 400 cm², more preferably from about 4cm² to about 100cm².

Preferably, the wound dressing further comprises a backing sheet extending over the active layer opposite to the wound facing side of the active layer. Preferably, the backing sheet is larger than the active layer such that a marginal region of width 1 mm to 50 mm, preferably 5mm to 20mm extends around the active layer to form a so-called island dressing. In such cases, the backing sheet is preferably coated with a pressure sensitive medical grade adhesive in at least its marginal region.

Preferably, the backing sheet is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers. It has been found that such moisture vapor transmission rates allow the wound under the dressing to heal under moist conditions without causing the skin surrounding the wound to macerate.

In a second aspect, the present invention provides a wound dressing comprising an antimicrobial composition according to the present invention.

The wound dressing is preferably in sheet form and comprises an active layer of the material according to the invention. The active layer would normally be the wound contacting layer in use, but in some embodiments it could be separated from the wound by a liquid-permeable top sheet. Preferably, the area of the active layer is from about 1cm² to about 400 cm², more preferably from about 4cm² to about 100cm².

Preferably, the wound dressing further comprises a backing sheet extending over the active layer opposite to the wound facing side of the active layer. Preferably, the backing sheet is larger than the active layer such that a marginal region of width 1 mm to 50 mm, preferably 5mm to 20mm extends around the active layer to form a so-called island dressing. In such cases, the backing sheet is preferably coated with a pressure sensitive medical grade adhesive in at least its marginal region.

Preferably, the backing sheet is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers. It has been found that such moisture vapor transmission rates allow the wound under the dressing to heal under moist conditions without causing the skin surrounding the wound to macerate.

The wound facing surface of the dressing is preferably protected by a removable cover sheet. The cover sheet is normally formed from flexible thermoplastic material. Suitable materials include polyesters and polyolefins. Preferably, the adhesive- facing surface of the cover sheet is a release surface. That is to say, a surface that is only weakly adherent to the active layer and the adhesive on the backing sheet to assist peeling of the adhesive layer from the cover sheet. For example, the cover sheet may be formed from a non-adherent plastic such as a fluoropolymer, or it may be provided with a release coating such as a silicone or fluoropolymer release coating.

The compositions and dressings according to the present invention are preferably sterile and packaged in a microorganism-impermeable container. Preferably, the sterility assurance level is better than 10⁻⁶. Preferably, the dressing has been sterilized by gamma-irradiation.

The compositions and dressings according to the present invention may further comprise therapeutically effective metal ions other than silver, for example bismuth, copper, nickel, zinc, manganese, magnesium, gold, or mixtures thereof. Preferably, the amounts of such metals are from 0.001 to 10wt.% of the composition or dressing, more preferably from 0.01 to 1wt.% of the composition or dressing. Preferably, the amounts of said other metals is from 10 to 10000ppm, more preferably from about 50 to about 1000ppm in the composition or dressing.

Preferably, the wound dressings according to the present invention are suitable for application directly to a wound surface.

In a further aspect, the present invention provides the use of a composition according to the present invention for the preparation of a dressing for the treatment of wounds. Preferably, the medicament is a wound dressing according to the present invention. Preferably, the treatment comprises applying the dressing directly to the surface of the wound.

The wound facing surface of the dressing is preferably protected by a removable cover sheet. The cover sheet is normally formed from flexible thermoplastic material. Suitable materials include polyesters and polyolefins. Preferably, the adhesive- facing surface of the cover sheet is a release surface. That is to say, a surface that is only weakly adherent to the active layer and the adhesive on the backing sheet to assist peeling of the adhesive layer from the cover sheet. For example, the cover sheet may be formed from a non-adherent plastic such as a fluoropolymer, or it may be provided with a release coating such as a silicone or fluoropolymer release coating.

The compositions and dressings according to the present invention are preferably sterile and packaged in a microorganism-impermeable container. Preferably, the sterility assurance level is better than 10⁻⁶. Preferably, the dressing has been sterilized by gamma-irradiation.

The compositions and dressings according to the present invention may further comprise therapeutically effective metal ions other than silver, for example bismuth, copper, nickel, zinc, manganese, magnesium, gold, or mixtures thereof. Preferably, the amounts of such metals are from 0.001 to 10wt.% of the composition or dressing, more preferably from 0.01 to 1wt.% of the composition or dressing. Preferably, the amounts of said other metals is from 10 to 10000ppm, more preferably from about 50 to about 1000ppm in the composition or dressing.

Preferably, the wound dressings according to the present invention are suitable for application directly to a wound surface.

In a further aspect, the present invention provides the use of a composition according to the present invention for the preparation of a dressing for the treatment of wounds. Preferably, the medicament is a wound dressing according to the present invention. Preferably, the treatment comprises applying the dressing directly to the surface of the wound.

Specific embodiments of the invention will now be described further, for the purpose of illustration, in the following examples:

### Reference Example 1

A zone of inhibition method was used to assess the antimicrobial activity of commercially available silver-containing dressings. Discs (6mm) from the impregnated barrier preparations (Acticoat®, Hydrophilic Wound Dressing, Arglaes®, Contreet®-H and Silverlon®) were prepared and placed on lawns of test bacteria that had been seeded onto Oxoid® nutrient agar. Zones of bacterial inhibition were measured daily during the first five days of incubation. Each experiment was performed in triplicate and the zone sizes reported as averages.

Resistance was observed visually by noting growth of individual colonies in the otherwise clear zones of inhibition around the disks. In other cases, resistance was seen to take the form of enhanced growth at the edge of the zone.

Experiments were performed with bacteria obtained from the American Type Culture Collection or from University sources. Strains of bacteria assessed in these studies included *Staphylococcus aureus* (ATCC 13709), *Staphylococcus epidermidis* (various CNS strains), *Klebsiella pneumoniae* (ATCC 10031), *Candida albicans* (ATCC 10231), *Escherichia coli* (ATCC 9637 and AG100), *Pseudomonas aeruginosa* (ATCC 27853 and various PSI strains) and a hospital-derived multiple drug resistant strain of *Staphylococcus aureus* (provided by the Dept of Clinical Microbiology and Pathology) and *Vancomycin resistant Enterococci* (VRE F23232, VRE F23245 and VRE 4030101).

It was found that none of the silver containing dressings was effective against *E*. *coli* AG100), *E.coli* ATCC9637 and *K. pneumoinae* ATCC 10031).

The yeast *C*. *albicans* was found to become resistant to silver, in the absence of inhibitor molecules, in almost all cases. This species therefore appears to be a suitable test model for assaying the ability of test molecules to overcome silver resistance.

Silver nitrate and silver sulfadiazine were also examined briefly for comparative purposes. They were inactive against *Escherichia coli* and *Klebsiella pneumoniae.* Activity was seen against *Staphylococcus aureus, Salmonella choleraesuis, Pseudomonas aeruginosa* and *Candida albicans.* Resistance was seen with *Candida albicans.*

### Example 1 (not part of the invention)

Tocopherol (Vitamin E) is a powerful antioxidant, neutralizes unstable free radicals, is lipid soluble and exerts its effects by stabilizing biological membranes. As a result of these properties it is implicated in the prevention of a wide range of degenerative diseases including heart disease, cancer, senility and diabetes.

Tocopherol was introduced into each dressing by adding 5ml of a 10µg/ml stock solution. The effect of these treatments on silver resistant colonies was assessed, and the results are summarised in Table 1.

The results demonstrate that tocopherol has no inherent antimicrobial activity, although in one sample some antimicrobial activity against *M*. *Smegmatis* was observed.

The results also demonstrate that Tocopherol overcomes silver resistance in some bacterial species. Tocopherol reversed silver resistance of *C*. *albicans* to silver derived from Acticoat, Silverlon and Contreet H in all cases. Tocopherol increased the size of the zones of inhibition against *S. choleraesuis* and *P. aeruginosa* in all dressing. This observation suggests Tocopherol may increase the sensitivity of bacteria to silver, and would be consistent with the molecule having properties that could be used to overcome silver resistance. However, tocopherol was ineffective at eliminating the ability of S. *aureus* and *M*. *smegmatis* to become resistant to silver.

### Example 2 (not part of the invention)

Resveratrol (trans-3, 5,4'-trihydroxystilbene) is a compound found largely in the skins of red grapes and has historically been used in oriental medicine used to treat diseases of the blood vessels and heart disease. It has antioxidative properties and has been shown to prevent or reduce tumour growth in animal studies by a mechanism involving the inhibition of cyclooxygenase-1 (COX-1), an enzyme that converts arachidonic acid to pro-inflammatory substances that stimulate tumor-cell growth.

Resveratrol was infused into each dressing by adding 5ml of a 10µg/ml stock solution. The effect of these treatments on silver resistant colonies was assessed, and is summarised in Table 2:

It can be seen that Resveratrol demonstrated the same pattern as Tocopherol on resistance. It had no inherent antimicrobial activity, almost completely reversed silver resistance against *C*. *albicans,* and appeared to increase the sensitivity of *S. choleraesuis* and *P*. *aeruginosa* to silver (as seen by an increase in the zones of inhibition). However as with Tocopherol, Resveratrol did not eliminate and in some cases appeared to promote the ability of *S*. *aureus* and *M. smegmatis to* become resistant to silver.

### Example 3 (not part of the invention)

Myristic acid is a natural fatty acid that is contained in plant and animal fats and that is also used as an additive in many foods.

Myristic acid was embedded into each dressing by adding 5ml of a 10µg/ml stock solution. The effect of these treatments on silver resistant colonies was assessed, and the results are summarised in Table 3.

The results show that Myristic acid has no inherent antimicrobial activity against any of the strains tested. It did not appear to increase the zones of inhibition when added to silver containing dressings in the absence of obvious signs of silver resistance. However, in four cases were silver resistance was observed against the dressing alone *(C. albicans* against Acticoat, Silverlon and Contreet-H and *S*. *aureus* against Contreet-H). This data is consistent with that for fusaric acid, and suggests that this molecules may be particularly useful at overcoming silver resistance.

### Example 4 (not part of the invention)

Green Tea (Pharmanex®) is present in green tea and is a strong antioxidant. It is sold by a variety of companies as a substance, which will protect against disease by a mechanism involving scavenging of free radicals.

Green Tea (Pharmanex®) was embedded into each dressing by adding 5ml of a 10µg/ml stock solution. The effect of these treatments on silver resistant colonies was assessed, and are summarised in Table 4.

The green tea extract was found to have very unique properties. Although, in five out of eight cases it had no antimicrobial properties on its own it did show antimicrobial activity against *S*. *aureus, M. smegmatis* and *C. albicans.* This made interpretation of the results very difficult as the apparent ability of this molecule to help *C*. *albicans* overcome silver resistance can partly be explained by its inherent antimicrobial activity against this species. However, one interesting property of this molecule is that although silver or Pharmanex on its own had no antibacterial activity, in this test system, the combination of these molecules appeared to promote antibacterial activity against *K*. *pneumoniae.*

### Example 5 (not part of the invention)

Curcumin is any anticancer drug that exerts its effects by inhibiting protein kinases involved in the G2 phase of the cell cycle. It is also known that it provides a large number of molecules (e.g. pesticides) from gaining entry to cells.

Curcumin was embedded into each dressing by adding 5ml of a 10µg/ml stock solution. The effect of these treatments on silver resistant colonies was assessed, and the results are shown in Table 5.

The results demonstrate that this molecule showed antimicrobial activity against *S*. *aureus, M. Smegmatis,* and *C*. *albicans.* These observations demonstrate that curcumin has antimicrobial properties in its own right, and although some synergic effects were observed with silver containing dressings against *C*. *albacans,* this was unlikely due to a mechanism involving the reversal of resistance to silver. It is likely that the pharmaceutical effects, or curcumin, may explain the antimicrobial properties observed here.

The inherent antimicrobial effects of curcumin suggest that this molecule is not suitable for use as a molecule that could increase the sensitivity of bacterial cells to silver or reverse silver resistance.

### Example 6 (not part of the invention)

Ellagic acid is a compound found in many fruits such as raspberries. It is reported to inhibit mutations in bacteria and was therefore tested as a potential molecule that would inhibit changed at the molecular (DNA) level that would cause the bacteria to become resistant to silver. However, this molecule was shown to have inherent antimicrobial properties. It did have synergistic effects with silver; however, due to its own antimicrobial properties it was impossible to ascertain the mechanism of this silver resistance.

### Example 7

Fusaric acid is a mycotoxin produced by certain molds and fungi. It is known to be a dopamine beta-hydroxylase inhibitor. However, Fusaric acid itself is inactive against human pathogenic bacteria.

The effect of Fusaric acid on the development of silver resistance was studied by a method similar to that of Examples 1 and 2. 5µl of 10mg/ml solution was embedded on a 6mm disc of dressing. The diameter of the zone of inhibition was measured in mm after incubation for 5 days at 37°C the number of resistant colonies within the Zone of Inhibition was recorded as RC values. R%+ approximate of the area of inhibition covered by resistant colonies. In the fusaric acid studies, five clinically-derived multidrug-resistant strains of *S*. *epidermidis* were also used. Fusaric acid was inactive by itself against these strains but resistance development/emergence was not seen when fusaric acid was combined with silver preparations. Similar results were seen with five clinically-derived multidrug-resistant strains of *Ps. aeruginosa.* The data obtained for Fusaric acid are shown in Table 6.

The above examples demonstrate that Tocopherol (vitamin E), Resveratrol and Myristic acid have an ability to reverse the silver resistance exhibited by C. *albicans.* In addition, Tocopherol and Resveratrol appear to increase the sensitivity of some types of bacteria to silver but have no effect on the silver sensitivity of other species. Both these molecules were found to be ineffective at reversing silver resistance exhibited by *S. aureus* and *M. smegmatis,* suggesting that both these molecules do not have a universal ability to reverse silver resistance. In contrast, although myristic acid did not increase the size of the zones of inhibition, when applied to dressings of wild type bacteria, it did reverse silver resistance in all four instances when silver resistance was observed. Fusaric acid is a particularly promising compound for the inhibition of silver resistance in a range of pathogenic microorganisms.

**Table 1**

| **Strains** | **Tocopher ol** | **Acticoat** | **Tocopherol + Acticoat** | **Silverlon** | **Tocopherol + Silverlon** | **Contreet-H** | **Tocopherol + Contreet-H** |
|---|---|---|---|---|---|---|---|
| S. aureus ATCC 13709 | 0 mm | 15 mm | 15 mm RC=8 | 13 mm | 12 mm RC=4 | 18 mm R=20% | 20 mm RC=14 |
| S. choleraesuis ATCC 9184 | 0 mm | 12 mm | 15 mm | 11 mm | 12 mm | 14 mm | 15 mm |
| P. aeruginosa ATCC 27853 | 0 mm | 11 mm | 16 mm | 11 mm | 12 mm | 13 mm | 15 mm |
| M. *smegmatis* ATCC 607 | 7 mm | 14 mm | 12 mm R=20% | 8 mm | 8 mm | 18 mm | 13 mm R=15% |
| C. *albicans* ATCC 10231 | 0 mm | 13 mm R=20% | 23 mm | 12 mm R=30% | 15 mm | 16 mm R=50% | 30 mm |

**Table 2**

| **Strains** | **Resveratrol** | **Acticoat** | **Resveratrol + Acticoat** | **Silverlon** | **Resveratrol + Silverlon** | **Contreet -H** | **Resveratrol + Contreet-H** |
|---|---|---|---|---|---|---|---|
| S. aureus ATCC 13709 | 0 mm | 15 mm | 18 mm RC=13 | 13 mm | 15 mm RC=11 | 18 mm R=20% | 23 mm R=3% |
| S. choleraesuis ATCC 9184 | 0 mm | 12 mm | 13 mm | 11 mm | 12 mm | 14 mm | 16 mm |
| P. aeruginosa ATCC 27853 | 0 mm | 11 mm | 16 mm | 11 mm | 12 mm | 13 mm | 16 mm |
| *M*. *smegmatis* ATCC 607 | 0 mm | 14 mm | 16 mm R=1 % | 8 mm | 13 mm RC=10 | 18 mm | 20 mm R=1 % |
| *C. albicans* ATCC 10231 | 0 mm | 13 mm R=20% | 27 mm R=1 % | 12 mm R=30% | 21 mm R=3% | 16 mm R=50% | 30 mm R=5% |

**Table 3**

| **Strains** | **Myristic Acid** | **Acticoat** | **Myristic Acid + Acticoat** | **Silverlon** | **Myristic Acid + Silverlon** | **Contreet -H** | **Myristic Acid + Contreet-H** |
|---|---|---|---|---|---|---|---|
| S. aureus ATCC 13709 | 0 mm | 15 mm | 13 mm | 13 mm | 10 mm | 18 mm R=20% | 19 mm |
| S. choleraesuis ATCC 9184 | 0 mm | 12 mm | 13 mm | 11 mm | 11 mm | 14 mm | 14 mm |
| P. aeruginosa ATCC 27853 | 0 mm | 11 mm | 14 mm | 11 mm | 11 mm | 13 mm | 14 mm |
| M. *smegmatis* ATCC 607 | 0 mm | 14 mm | 10 mm | 8 mm | 10 mm | 18 mm | 14 mm |
| C. albicans ATCC 10231 | 0 mm | 13 mm R=20% | 17 mm | 12 mm R=30% | 17 mm | 16 mm R=50% | 22 mm |

**Table 4**

| **Strains** | **Green Tea** | **Acticoat** | **Green Tea + Acticoat** | **Silverlon** | **Green Tea + Silverlon** | **Contreet -H** | **Green Tea + Contreet-H** |
|---|---|---|---|---|---|---|---|
| S. aureus ATCC 13709 | 10 mm | 15 mm | 16 mm | 13 mm | 13 mm | 18 mm R=20% | 17 mm |
| S. choleraesuis ATCC 9184 | 0 mm | 12 mm | 14 mm | 11 mm | 11 mm | 14 mm | 16 mm |
| P. aeruginosa ATCC 27853 | 0 mm | 11 mm | 13 mm | 11 mm | 12 mm | 13 mm | 15 mm |
| *K. pneumoinae* ATCC 10031 | 0 mm | 0 mm | 7 mm | 0 mm | 7 mm | 0 mm | 0 mm |
| *M. smegmatis* ATCC 607 | 8 mm R=20% | 14 mm | 12 mm R=15% | 8 mm | 9 mm | 18 mm | 13 mm R=15% |
| *C. albicans* ATCC 10231 | 16 mm | 13 mm R=20% | 23 mm | 12 mm R=30% | 18 mm | 16 mm R=50% | 24 mm |

**Table 5**

| **Strains** | **Curcumin** | **Acticoat** | **Curcumin + Acticoat** | **Silverlon** | **Curcumin + Silverlon** | **Contreet -H** | **Curcumin + Contreet-H** |
|---|---|---|---|---|---|---|---|
| S. *aureus* ATCC 13709 | 9 | 15 | 13 | 13 | 13 | 18 R=20% | 19 |
| *S. choleraesuis* ATCC 9184 | 0 | 12 | 13 | 11 | 11 | 14 | 14 |
| *P. aeruginosa* ATCC 27853 | 0 | 11 | 13 | 11 | 12 | 13 | 16 |
| M. smegmatis ATCC 607 | 8 | 14 | 11 | 8 | 9 | 18 | 12 |
| C. albicans ATCC 10231 | 9 | 13 R=20% | 18 | 12 R=30% | 15 | 16 R=50% | 23 |

**Table 6**

| **Strains** | **Fusaric Acid** | **Acticoat** | **Fusaric acid + Acticoat** | **Silverlon** | **Fusaric Acid + Silverlon** | **Contreet-H** | **Fusaric acid +Contreet-H** |
|---|---|---|---|---|---|---|---|
| E.coli AG100 | 0 mm | 0 mm | 11 mm | 0 mm | 8 mm | 0 | 0 |
| S. choleraesuis ATCC 9184 | 0 mm | 12 mm | 13 mm | 11 mm | 12 mm | 14 mm | 16 mm |
| P. aeruginosa PSA083349-1 | 0 mm | 14 mm | 17 mm | 25 mm | 25 mm | 12 mm | 12 mm |
| P, aeruginosa PSA084459 | 0 mm | 12 R=50mm | 13 mm | 9 mm | 11 mm | 11 mm | 14 mm |
| P. aeruginosa PSA083367-1-1 | 0 mm | 9mm | 17 mm | 8 | 17 mm | 11mm | 14 mm |
| P.aeruginosa | 0 | 12mm R=50% | 14mm | 9mm | 10mm | 12 | 16 mm (R=15%) |

## Claims

1. An antimicrobial composition comprising silver and fusaric acid which interacts with a microbial cell wall to inhibit microbial silver resistance.

2. An antimicrobial composition according to claim 1, wherein the silver comprises metallic silver.

3. An antimicrobial composition according to any preceding claim, wherein the silver is applied to a solid support material.

4. An antimicrobial composition according to any preceding claim, wherein the composition comprises from about 0.01 wt.% to about 5wt.% of silver, based on the dry weight of the composition.

5. An antimicrobial composition according to any preceding claim, wherein the composition comprises from about 0.01 wt.% to about 5wt.% of the one or more resistance inhibiting compounds, based on the dry weight of the composition.

6. An antimicrobial composition according to any preceding claim, comprising a solid substrate material incorporating the silver and the resistance inhibiting compound.

7. An antimicrobial composition according to claim 6, wherein the substrate is a woven fabric, a knitted fabric, a nonwoven fabric, or a freeze-dried or solvent-dried sponge.

8. An antimicrobial composition according to claim 6, wherein the substrate comprises charcoal.

9. An antimicrobial composition according to claim 6, wherein the substrate comprises a material selected from the group consisting of oxidized regenerated cellulose, collagen, chitosan, alginates, and mixtures thereof

10. An antimicrobial composition according to any preceding claim, which is sterile and packaged in a microorganism-impermeable container.

11. A wound dressing comprising an antimicrobial composition according to any one of claims 1 to 10.

12. A wound dressing according to claim 11, which is sterile and packaged in a microorganism-impermeable container.

## Patentansprüche

1. Antimikrobielle Zusammensetzung, umfassend Silber und Fusarinsäure, die mit einer mikrobiellen Zellwand in Wechselwirkung tritt, um mikrobielle Silberresistenz zu hemmen.

2. Antimikrobielle Zusammensetzung nach Anspruch 1, wobei das Silber metallisches Silber umfasst.

3. Antimikrobielle Zusammensetzung nach einem vorangehenden Anspruch, wobei das Silber auf ein festes Trägermaterial aufgebracht ist.

4. Antimikrobielle Zusammensetzung nach einem vorangehenden Anspruch, wobei die Zusammensetzung von etwa 0,01 Gew.-% bis etwa 5 Gew.-% Silber, bezogen auf das Trockengewicht der Zusammensetzung, umfasst.

5. Antimikrobielle Zusammensetzung nach einem vorangehenden Anspruch, wobei die Zusammensetzung von etwa 0,01 Gew.-% bis etwa 5 Gew.-% des einen oder der mehreren resistenzhemmenden Verbindungen umfasst, bezogen auf das Trockengewicht der Zusammensetzung.

6. Antimikrobielle Zusammensetzung nach einem vorangehenden Anspruch, die ein festes Substratmaterial umfasst, welches das Silber und die resistenzhemmende Verbindung enthält.

7. Antimikrobielle Zusammensetzung nach Anspruch 6, wobei das Substrat ein gewebter Stoff, ein gewirkter Stoff, ein Vliesstoff oder ein gefriergetrockneter oder lösemittelgetrockneter Schwamm ist.

8. Antimikrobielle Zusammensetzung nach Anspruch 6, wobei das Substrat Holzkohle umfasst.

9. Antimikrobielle Zusammensetzung nach Anspruch 6, wobei das Substrat ein Material umfasst, das ausgewählt ist aus der Gruppe, bestehend aus oxidierter regenerierter Cellulose, Collagen, Chitosan, Alginaten und Mischungen davon.

10. Antimikrobielle Zusammensetzung nach einem vorangehenden Anspruch, die steril und in einem für Mikroorganismen undurchlässigen Behälter verpackt ist.

11. Wundverband, der eine antimikrobielle Zusammensetzung nach einem der Ansprüche 1 bis 10 umfasst.

12. Wundverband nach Anspruch 11, der steril und in einem für Mikroorganismen undurchlässigen Behälter verpackt ist.

## Revendications

1. Composition anti-microbienne comprenant de l'argent et de l'acide fusarique, qui interagit avec une paroi de cellule microbienne pour inhiber la résistance microbienne à l'argent.

2. Composition antimicrobienne selon la revendication 1, dans laquelle l'argent comprend l'argent métallique.

3. Composition antimicrobienne selon l'une quelconque des revendications précédentes, dans laquelle l'argent est appliqué sur un matériau de support solide.

4. Composition antimicrobienne selon l'une quelconque des revendications précédentes, la composition comprenant d'environ 0,01 % en poids à environ 5 % en poids d'argent sur la base du poids sec de la composition.

5. Composition antimicrobienne selon l'une quelconque des revendications précédentes, la composition comprenant d'environ 0,01 % en poids à environ 5 % en poids d'un ou plusieurs composés inhibant la résistance, sur la base du poids sec de la composition.

6. Composition antimicrobienne selon l'une quelconque des revendications précédentes, comprenant un matériau de substrat solide comprenant l'argent et le composé inhibant la résistance.

7. Composition antimicrobienne selon la revendication 6, dans laquelle le substrat est un tissu tissé, un tissu tricoté, un tissu non tissé ou une éponge lyophilisée ou séchée par un solvant.

8. Composition antimicrobienne selon la revendication 6, dans laquelle le substrat comprend le charbon.

9. Composition antimicrobienne selon la revendication 6, dans laquelle le substrat comprend un matériau choisi dans le groupe constitué de la cellulose régénérée oxydée, du collagène, du chitosan, des alginates et de leurs mélanges.

10. Composition antimicrobienne selon l'une quelconque des revendications précédentes, qui est stérile et emballée dans un conteneur imperméable aux microorganismes.

11. Pansement de plaie comprenant une composition antimicrobienne selon l'une quelconque des revendications 1 à 10.

12. Pansement de plaie selon la revendication 11, qui est stérile et emballé dans un conteneur imperméable aux microorganismes.
